Europäisches Patentamt

European Patent Office

Office européen des brevets

(11) Publication number: **0 258 441**
A1

# EUROPEAN PATENT APPLICATION

published in accordance with Art. 158(3) EPC

(21) Application number: 87900884.5

(22) Date of filing: 22.01.87

Data of the international application taken as a basis:

(86) International application number:
PCT/JP87/00038

(87) International publication number:
WO87/04458 (30.07.87 87/17)

(51) Int. Cl.³: **C 12 N 5/02**
//B01J13/00, C12N11/04

(30) Priority: 25.01.86 JP 14324/86
28.01.86 JP 17514/86

(43) Date of publication of application:
09.03.88 Bulletin 88/10

(84) Designated Contracting States:
CH DE FR GB LI NL

(71) Applicant: NITTA GELATIN INC.
55-1, Honmachi 2-chome Higashi-ku
Osaka-shi Osaka 541(JP)

(72) Inventor: KOEZUKA, Masahiro
412, Futamata Yao-shi
Osaka 581(JP)

(72) Inventor: HATA, Masataka
F-1004, 5, Misawacho Ibaraki-shi
Osaka 567(JP)

(72) Inventor: SUZUKI, Kaneo
167-11, Ishiharacho Kashihara-shi
Nara 634(JP)

(72) Inventor: YASUGI, Shigeo
1657-103, Sahodai 2-chome Nara-shi
Nara 630(JP)

(72) Inventor: ENAMI, Junpei
865, Oaza Kitakobayashi Mibu-machi
Shimotsuga-gun Tochigi 321-02(JP)

(74) Representative: Geering, Keith Edwin et al,
REDDIE & GROSE 16 Theobalds Road
London WC1X 8PL(GB)

(54) **PROCESS FOR CULTIVATING ANIMAL CELLS ON A LARGE SCALE.**

(57) A process for cultivating animal cells on a large scale, which comprises embedding animal cells in collagen gel and conducting cultivation of the animal cells while suspending the collagen gel in a culture medium. This process makes it possible to conduct cultivation with high cell density, cultivate cells which could not be conventionally cultivated, and conduct long-term cultivation. This large-scale cultivating process can be utilized for effectively collecting a large amount of an animal cell product.

Fig. 1

EP 0 258 441 A1

SPECIFICATION

A Method for Large-Scale Cultivation of Animal Cells

Technical Field

This invention is related to a method for cultivation of animal cells on a large scale.

Background Technology

Although some cells can grow free-floating in suspension culture, most animal cells grow only after attachment to suitable substrata. For this reason, cells have been cultured on solid substrata such as glass or plastic which have electric charges. Occasionally, these solid substrata have been coated with proteins such as collagen or fibronectin to promote cell attachment. Under these culture conditions, cells form monolayers and grow two-dimensionally on the substrata. For large-scale cultivation of cells in monolayers, it is necessary to increase the culture area. The following methods have been used for this purpose.

(1)  Large Culture Flask Method

This is a regular monolayer culture method. Cells are cultured stationally in monolayers in desired numbers of large culture flasks or Roux flasks.

(2)  Roller Bottle Method

Cells are cultured on the inner wall of cylindrical

- 1 -

bottles.  The bottles are rotated slowly.

(3)   Sterilin-type Culture Method

Cells are cultured on both sides of a corrugated polyester film which is concentrically wound.

(4)   Multilayer Plate Method

Cells are cultured on glass or polycarbonate plates stacked to form multilayers with spacing.

(5)   Multistage Monolayer Method

This is a modification of the multilayer plate method.  Stationary culture elements of a shallow box-type with the culture surface made of glass, polyester or polystyrene are stacked up.  Cells are cultured inside each element using only the gas phase in common.

(6)   Glass Fiber Column Method

Columns are filled with spirally wound glass fibers.  Cells are cultured on the surface of glass fibers while exchanging the culture medium.

(7)   Glass Bead Column Method

Columns are filled with glass beads.

Cells are cultured on the surface of glass beads while exchanging the culture medium.

(8)   Hollow Fiber Method

Columns are filled with bundles of hollow fibers.  Cells are cultured inside the hollow fibers.

(9)   Superbeads Method (or Microcarrier Method)

This is a combination of monolayer culture method and suspension culture method.  Cells are attached on the surface of tiny beads and the beads are maintained in suspension by slow stirring.

At present, the highest cell density can be achieved by the superbead method.  In this method, beads, to which cells are attached, are stirred in a culture bottle.  This causes the beads to collide against each other.  Thus, the cells on the beads become heavily damaged.

Many other large-scale cultivation methods have been proposed.  However, all of them commonly used are monolayer culture method.  Cells are cultured two-dimensionally on the surface of beads or on the surface of glass or plastic.  Since monolayer culture conditions are different from the conditions in vivo, long-term cultivation of freshly isolated cells is difficult.  Cells easily lose differentiated functions and cease growing.

In the light of these problems, it is the object of this invention to provide a method for large-scale cultivation of animal cells capable of cultivating to a higher density than in the conventional methods and also capable of cultivating for a long period.

## Disclosure of the Invention

In order to achieve the above-described object, this invention provides a method for large-scale cultivation of animal cells in which cells are embedded in collagen gels and the gels are suspended in a culture medium to cultivate the cells on a large scale.

This invention is described in detail below.

The method of embedding cells in collagen gel is as follow; the cells are dispersed in a collagen solution and the solution is then gelled. By this method, the animal cells are uniformly embedded in the collagen gel easily.

The purpose of embedding animal cells in collagen gel is to support the growth of anchorage-dependent cells and to mimic the _in vivo_ conditions so that the cells can grow three-dimensionally. By this embedding method, the cells are grown three-dimensionally, so that the cells can be cultivated to a higher density and for a longer period than in the conventional methods. It is further possible to induce cell differentiation. Besides, since cells are physically protected with collagen gel, the cells are hardly injured in suspension culture. This principle may also be applied to anchorage-independent cells.

- 4 -

The reason for using collagen gel as a substrate to support animal cell growth in this invention is because collagen is a fibrous protein occuring in every parts of the animal body and thus _in vivo_ environment can be reproduced _in vitro_ by collagen gel. The type of collagen is not particularly limited, and various types may be used. Since the collagen is in the gel form, exchange of substances between inside the gel (cells) and outside the gel (such as respiration, absorption of nutrition, excretion, and release of secretion) is possible. The shape of the collagen gel is not particularly limited. Any shape can be used as far as it is possible to be floated in culture medium. For example, shapes of grains, noodles or sheets are applicable. From the viewpoint of exchanging substances between inside and outside the collagen gel in a wider area, the granular shape is preferable. In this way, the exchange of substances between the cells and the outer environment may be made in an outstandingly wider area as compared with the conventional superbead method or the culture method on collagen gel surface. The shape of grains may be spherical, columnar, ellipsoidal, cubic, rectangular parallelepiped, irregular or any other, and is not particularly limited.

In the following description of this invention, therefore, to "obtain fragments" does not mean only to make into granular form, but is intended to mean noodle shape, sheet shape, or any other shape as described above.

There is no particular limitation to the means or method of the collagen gel preparation. For example, collagen solution in which animal cells are dispersed is put in a multiplate well and gelled, then the prepared collagen gels are taken out and placed into a culture medium, or collagen solution in which animal cells are dispersed is placed into a properly warmed culture medium to promote gelling, and after the gelation, the collagen gel is crushed into smaller pieces. In the former method, however, the process of taking out the collagen gels from the multiplate well is a manual work, and the efficiency may be inferior to the latter. In the latter method, on the other hand, it is not easy to control the collagen gel size or shape constantly.

When the collagen gel fragments are continuously obtained and supplied into a culture medium, the above worsening of efficiency may be prevented, and the collagen gel size and shape may be easily controlled.

As a method of obtaining continuously collagen

- 6 -

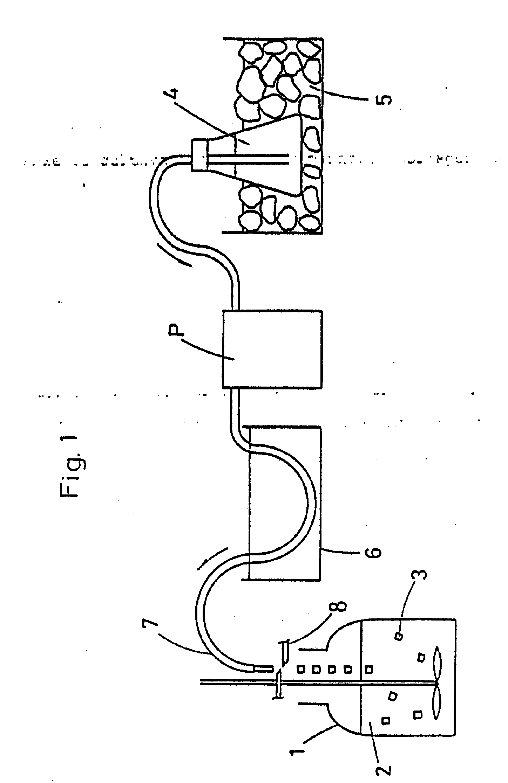

gel fragments, collagen solution in which animal cells are dispersed is continuously gelled, and cut into granular forms continuously, then these grains are placed into the culture medium. Fig. 1 shows an example of an apparatus used in the embodiment of the large-scale cultivation method of this invention. As shown in Fig. 1, a collagen solution (4) in which animal cells are dispersed is kept in an iced water (5) so as not to be gelled, and this collagen solution (4) is sent into a culture tank (1) by means of a pump (P), by way of an intervening hot water tank (6) at a proper temperature (such a temperature that collagen gels in a short time and that animal cells are not damaged, for example, around 37°C) while the collagen solution (4) is gelled. This collagen gel is extruded from a tube (7) cut by a cutting system (8), like cutter etc., and small pieces of the collagen gel (3) are dropped into the culture tank (1) filled with culture medium (2). When thus fragmented, it is easy to form the collagen gel pieces (3) in a uniform shape. When the collagen gel (3) is collected properly, the supply of the collagen solution is stopped, and the collagen gels (3) are suspended in the culture medium (2) to cultivate.

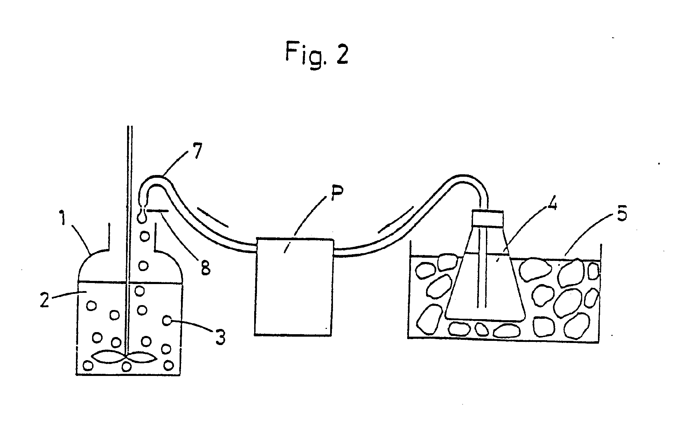

As another method, it is also possible to obtain continuously collagen gel fragments by dropping the collagen solution, in which animal cells are dispersed, continuously in liquid form and gelling the drops. Fig. 2 shows an example of the apparatus used in the embodiment of the large-scale cultivation method of this invention. As shown in Fig. 2, a collagen solution (4), in which animal cells are dispersed, is kept in an iced water (5) so as not to be gelled, and this collagen solution (4) is sent into a culture tank (1) by means of a pump (P). The collagen solution is dropped from a tube (7) into a culture medium (2) warmed at a proper temperature (preferably at 37°C considering the collagen gelling temperature and the culture conditions) and stirred. Or, the collagen solution (4) may be supplied into the culture medium (2) while cutting by a cutting system (8). In this way, the collagen gels in the culture medium (2), and the resulting collagen gels (3) float in the culture medium (2). When the collagen gels (3) are properly collected, the supply of the collagen solution is stopped, and cultivation is started while suspending the collagen gels (3) in the culture medium (2).

In the above-mentioned methods of obtaining collagen gel fragments, in order to enhance the gelling ability and to fragment into uniform shape, it may be necessary to properly adjust the temperature, viscosity, specific gravity, osmotic pressure, boundary surface tension and the like of the culture medium. The preparation methods are not particularly limited, but it is preferable to use a mixture of solution of derivatives from sugars and/or polysaccharides and culture medium, or a solution having a similar composition. For example, 1:1 mixture of culture medium and 0.25M sucrose, or culture medium containing 0.3% of methyl cellulose may be used. After the completion of gelling, the culture medium is returned to the original for cultivation.

In the apparatus shown in Fig. 2, it may be also possible to arrange as follows: first, the solution in the culture tank (1) possesses only the gel forming ability, and is prepared as a collagen gel forming fluid without cultivating ability. After the collagen solution dropped into the solution are gelled to form collagen gel, the collagen gel forming fluid is removed from this culture tank (1), and then a culture medium is charged in to start cultivation. Since the collagen is first gelled in the fluid excelling in collagen gel forming ability, an excellent collagen gel with uniform shape etc. is obtained by this method. Incidentally, it may be also possible to start cultivation after transferring the collagen gels obtained in the collagen gel forming fluid into other culture tank.

As in these examples, when the collagen solution, in which animal cells are dispersed, sent into the culture medium or into the collagen gel forming fluid through an enclosed passage such as the tube, the chances of contamination are decreased. By varying the tube size or the above-mentioned cutting intervals, the size of collagen gel may be freely changed. Besides, by changing the sectional shape of the tube, the shape of the collagen gel may be freely varied. It is possible to transfer this collagen solution in a short time and at high efficiency if multiple and/or large-capacity cultivation apparatuses are used. The method of the collagen solution supply is not limited to the above-mentioned three examples only.

The means of cultivation while suspending the collagen gel, which are embedded with animal cells, in a culture medium is likewise not limited. For example, the following methods may be considered.

(1)  Spinner method

Collagen gels embedded with animal cells and culture medium are placed in a spinner flask, and the culture medium is agitated by stirrer or the like to maintain the collagen gels in suspension.

(2)  Shaking culture flask method

Collagen gels embedded with animal cells and culture medium are placed in a shaking culture flask. The flask is shaken to stir the culture medium.  Thus, the collagen gels are maintained in suspension.

(3)  Roller bottle method

Collagen gels embedded with animal cells and culture medium are placed in a roller bottle.  The roller bottle is rotated to stir the culture medium. Thus, the collagen gels are maintained in suspension.

(4)  Perfusion culture method

Collagen gels embedded with animal cells are placed in a column, and culture medium is perfused in this column to feed the cells in the collagen gel.

(5)  Air agitation method

Collagen gels embedded with animal cells and culture medium are placed in an air agitator, and the culture medium is stirred by sending air stream, thereby the collagen gels are maintained in suspension.

In any one of the methods shown above, the efficiency will be enhanced when the method for obtaining the collagen gels embedded with animal cells and the method for suspending the collagen gels are carried out continuously.

Incidentally, the cultivation conditions will be improved when the culture medium is stirred while the collagen gels embedded with animal cells are maintained in suspension.

In this invention, animal cells are cultivated embedded in collagen gels. However, if collagen gels having ordinary gel strength are used, the collagen gels may shrink. This shrinking of the collagen gel is considered to be due to morphological change of cells in culture. Once it occurs, it may be impossible to encourage cell growth. Accordingly, in this invention, it is preferable to prevent shrinkage of collagen gels by using collagen gels having high gel strength (50 g or more). In this specification, the gel strength refers to the value measured in the method specified below.

(Method for measurement of gel strength)

To 24 ml of collagen solution, 3 ml of 0.1 M phosphate buffer solution (pH 7.4) containing 1.4 M sodium chloride and 3 ml of sodium hydroxide solution are added while cooling, and mixed well. After the final pH is adjusted to 7.4, gel is formed by heating at 37°C for one hour. The obtained gel is placed in a rheometer (LRM-2002D, made by Fudo Kogyo Co., Tokyo), and the gel strength is measured by using a 0.5 inch plunger. The conditions of measurement are insertion depth of 10 mm and insertion speed of 20 mm/min.

The method for large-scale cultivation of animal cells of this invention comprises large-scale cultivation of animal cells by suspending collagen gels, which are embedded with animal cells, in a culture medium, so that very high density cultures and a long-term culture can be performed.

Brief Description of the Drawings

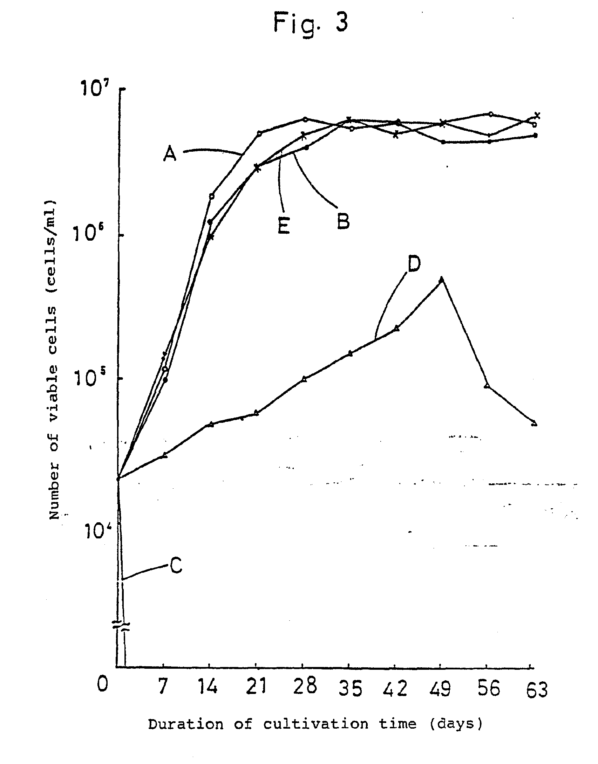

Fig. 1 and Fig. 2 are schematic illustrations of examples of the apparatuses used in the embodiments of this invention for cultivation by gelling the collagen solution, in which animal cells are dispersed, and suspending in the culture medium. Fig. 3 is a growth curve of the cells cultured under these conditions.

Mode for Carrying Out the Invention

Examples and reference examples are described below.

In the examples and reference examples, the number of viable cells was measured in the following method.

(Method for measurement of number of viable cells)

After collagen gels with cells are dissolved by collagenase treatment, the cells are collected by a

- 13 -

low speed centrifugation. The pelleted cells are dispersed in phosphate-buffered saline and mixed with 0.5% trypan blue in phosphate-buffered saline at a ratio of 1:2. Dead cells are stained blue by the dye. Undyed cells are counted as viable cells and calculated in a hemocytometer according to the conventional method.

(Example 1)

Rat tail tendon that had been thoroughly washed, defatted and decalcified was stirred in a hydrochloric acid solution (pH 2.5) for 24 hours at 4°C, and collagen was extracted. Insoluble residues were removed from this extract by centrifugation, and sodium hydroxide solution was added to adjust the pH to 7.0. The solution was let stand overnight, and the precipitate was collected by centrifugation. The pellet was dissolved again in hydrochloric acid solution (pH 3.0), and again precipitated as above. After thorough washing in water, the precipitate was finally dissolved in hydrochloric acid solution at pH 3.0. A purified acid-soluble rat tail tendon collagen solution with a concentration of 3.0 mg/ml was thus obtained. This collagen solution was sterilized by ultraviolet irradiation.

To 8 parts by volume of the 3.0 mg/ml acid-soluble collagen solution, 1 part by volume of 10 times concentrated Ham's F12 medium (not containing $NaHCO_3$), and 1 part by volume of a solution containing 2.2% $NaHCO_3$, 0.05N NaOH and 0.2M HEPES were added. Tumor cells dissociated from transplanted dural sarcoma were added to the mixture and mixed well. Thus, a collagen solution (mixed solution) with dispersed animal cells was prepared. This collagen solution was kept in an ice-water bath (4°C) until use to prevent gelling.

This collagen solution (mixed solution) (4) was sent into a culture tank (capacity: 3 liters; manufactured by Techne Co.) (1) through a pump (P) as shown in Fig. 1, by way of an intervening 37°C hot water tank (6) to gel the collagen solution (4). This collagen gel was extruded through a tube (7), cut into columnar shape (8 mm diameter x 10 mm length) by means of a cutter (8), and dropped into the culture tank (1) containing 1 liter of culture medium (2). The gel strength of this collagen gel was 200 g. The pump used was a roller pump (RP) (made by Tokyo Rika Co.), and the flow rate was 1.5 ml/min. The tube used to transfer the collagen solution was a silicone tube with an inside diameter of 8 mm. The pump was stopped when the collagen gel quantity in the culture medium reached 1 liter to stop supply of the collagen solution. Immediately, the stirrer was rotated to agitate the culture medium, and the culture was started by suspending the collagen gel in the medium. The culture medium was changed every 24 hours.

The changes in the number of viable cells in relation to the duration of cultivation time are indicated by curve A in Fig. 3.

(Example 2)

The same collagen solution (mixed solution) (4) with dispersed animal cells as prepared in Example 1 was used. The same pump (P) and silicone tube (7) as used in Example 1 were used. As shown in Fig. 2, while keeping the collagen solution (4) at 4°C, it was intermittently sent into the culture tank (1) (capacity: 3 liters; manufactured by Techne Co.) by using a foot switch. A total of one liter of this collagen solution (4) was extruded from a narrow end of the tube (7) and dropped intermittently into the culture medium warmed at 37°C (2) by means of cutter (8) to be gelled in an ellipsoidal form (diameter: 5 to 15 mm). The culture medium (2) was a 1:1 mixture of the same culture medium as used in Example 1 and 0.25M sucrose. Collagen gel was similarly formed when used a culture medium prepared by dissolving methyl cellulose in the medium used in Example 1 at a concentration of 0.3%.

Subsequently, after removing the culture medium (2) by a suction pump, one liter of the same culture medium as used in Example 1 was poured in the culture tank (1), and the collagen gels (3) were suspended in the culture medium to start cultivation. The culture medium was changed every 24 hours.

Changes in the number of viable cells in relation to the duration of cultivation time are shown by curve B in Fig. 3.

(Example 3)

The same collagen solution (mixed solution) with dispersed animal cells as prepared in Example 1 was placed into a multiplate (16 mm diamter, 24 wells) by 3 ml per well, and warmed to 37°C. The collagen solution gelled in several minutes, and animal cells were embedded in the collagen gel. The shape of each collagen gel was a columnar form measuring 1.6 cm in diameter and 1.5 cm in height. The gel strength of the collagen gel was 200 g. One hundred pieces (300 ml) of the collagen gel were taken out by tweezers and placed into a 1-liter spinner flask. To this flask, 300 ml of the same culture medium as used in Example 1 was poured. The culture medium was agitated by a stirrer to suspend the collagen gels, and the cultivation was started. The culture medium was changed every 24 hours.

Changes in the number of viable cell in relation to the duration of cultivation time are shown by curve E in Fig. 3.

(Example 4)

Three-hundred ml of the same collagen solution (mixed solution) with dispersed animal cells as prepared in Example 1 were poured into 300 ml of the same culture medium as used in Example 1 which was prewarmed to 37°C. The collagen solution was gelled. The obtained collagen gel was crushed by rotating the rotary blades of propeller type stirrer. The diameter of the crushed collagen gel pieces ranged from a minimum of 3 mm to a maximum of 30 mm, and were irregular in shape. By rotating the stirrer, the culture medium was stirred, and the crushed collagen gels were suspended to cultivate the animal cells. The culture medium was changed every 24 hours.

In this large-scale cultivation, the number of viable cells on the 21st day of cultivation was $2 \times 10^{6}$ cells/ml.

(Reference example 1)

The same animal cells as used in Example 1 were attached to the surface of microcarriers having diameters of 100 to 200 μm (Cytodex 3; Pharmacia Fine Chemicals Co.). Five to ten cells were present per microcarrier. These microcarriers corresponding to 1.5 g in dry weight was placed into the same 3-liter culture tank as used in Example 1, and 1 liter of the same culture medium as used in Example 1 was placed in the tank to start cultivation. All the cells died within a day. Thus, the cultivation was unsuccessful.

Changes in the number of viable cells in relation to the duration of cultivation time are indicated by curve C in Fig. 3.

(Reference example 2)

One ml of the same collagen solution (mixed solution) with dispersed animal cells as prepared in Example 1 was placed in each well (1.6 cm in diameter) of a multiplate (24 wells) and warmed to 37°C to form gels. The each collagen gel was overlayed with 1 ml of the same culture medium as used in Example 1 to start cultivation. The culture medium was changed every 24 hours.

Changes in the number of viable cells in relation to the duration of cultivation time are shown by curve D in Fig. 3.

Considering from the results in Examples 1 to 4 and Reference example 1, it is clear that the cells that cannot be cultivated by the superbead method, which is capable of cultivating cells to the highest density among the conventional large-scale cultivation methods, can be cultivated at a higher density and for a longer period by the large-scale cultivation method of this invention.

As shown in Reference example 2, in the stationary collagen gel cultivation method, in which collagen gel was embedded with animal cells and overlayed with culture medium, the cell density was not as high as in the large-scale cultivation method of this invention as shown in Fig. 3, and the number of cells decreased as the cultivation prolonged.

In Example 3, manual labor was needed to take out the collagen gel from the multiplate and put into the culture. By contrast, in Examples 1 and 2, the whole process was done by an operation of the pump to put the collagen gel into the culture tank and the quantity of collagen gel can be adjusted by the timing of stopping the pump or detaching the tube. In Example 1, the cultivation could be immediately started by feeding collagen gel. In Example 2, by removing by means of a suction pump the culture medium used for gel formation and replacing it with the original culture medium, the cultivation could be started immediately.

Thus, according to the large-scale cultivation method of animal cells of this invention, it is possible to cultivate the cells for a longer period as compared with the conventional large-scale cultivation methods. Cells that cannot be grown by the conventional large-scale cultivation methods can be successfully grown. It was also disclosed that the efficiency is high as the collagen gels embedded with animal cells are supplied into the culture medium continuously. In addition, the chances of contamination are decreased when the collagen solution with dispersed animal cells is sent into the culture medium through an enclosed passage such as a tube. Moreover, by properly varying the tube size or cutting intervals, the size of the collagen gel embedded with animal cells can be easily changed.

Meanwhile, the large-scale cultivation method of animal cells of this invention is not limited to the above examples.

Industrial Applicability

By the method for large-scale cultivation method of animal cells of this invention, it is possible to cultivate even the cells that cannot be grown at all by the conventional superbead method in a large quantity. The cell density attained by the method of this invention is higher than that of the superbead method which is known as the large-scale cultivation method with the highest density known at the present. Therefore, by the large-scale cultivation method of this invention, a large quantity of cells that could not be cultivated by the conventional superbead method can be cultivated efficiently. In addition, since the cell differentiation is also known to be promoted by the collagen gel cultivation method, a large quantity of cellular products (or secreted products) can be collected efficiently.

The above cell collection can be done, for example, by the following method.

(Method for Cell Collection)

Collagen gels containing cells are cut into square pieces of about 3 mm by means of scissors or the like, and collagenase is added to a final concentration of 0.02% to dissolve the collagen gel. After the collagen gel is dissolved, the cells are collected by low-speed centrifugation.

The method for large-scale cultivation of this invention may be applied to any animals cells, whether of anchorage-dependent cells or anchorage-independent cells. In particular, when applied in cultivation of anchorage-dependent cells, its effect is outstanding.

Various products can be obtained, including vaccines, enzymes, hormones, antibodies, interferon, and nucleic acids, etc.

- 23 -

## CLAIMS

1. A method for large-scale cultivation of animal cells for cultivating animal cells in a large quantity by embedding cells in collagen gels and cultivating in suspension.

2. The method for large-scale cultivation of animal cells as defined in claim 1, wherein the animal cells are embedded in the collagen gels by dispersing the cells in a collagen solution and gelling the collagen solution.

3. The method for large-scale cultivation of animal cells as defined in claim 1, wherein the collagen gel fragments in which animal cells are dispersed are continuously obtained and placed into the culture medium.

4. The method for large-scale cultivation of animal cells as defined in claim 3, wherein the animal cells are embedded in the collagen gels by dispersing the animal cells in a collagen solution and gelling the collagen solution.

5. The method for large-scale cultivation of animal cells as defined in claim 4, wherein the collagen gel fragments in which animal cells are dispersed are continuously obtained by continuously gelling the collagen solution in which animal cells are dispersed, and cutting the collagen gels into smaller pieces.

6. The method for large-scale cultivation of animal cells as defined in claim 4, wherein the collagen gel fragments in which animal cells are dispersed are continuously obtained by continuously dropping the collagen solution, in which the animal cells are dispersed, into a prewarmed medium to form gels.

7. The method for large-scale cultivation of animal cells as defined in claim 6, wherein gelling is affected by the nature of culture medium.

8. The method for large-scale cultivation of animal cells as defined in claim 6, wherein gelling is affected by a collagen gel forming solution.

Fig. 1

Fig. 2

0258441

# Fig. 3

Duration of cultivation time (days)

# INTERNATIONAL SEARCH REPORT

International Application No `PCT/JP87/00038`

## I. CLASSIFICATION OF SUBJECT MATTER (if several classification symbols apply, indicate all) [3]

According to International Patent Classification (IPC) or to both National Classification and IPC

Int.Cl[4]    C12N5/02//B01J13/00, C12N11/04

## II. FIELDS SEARCHED

| Minimum Documentation Searched [4] | |
|---|---|
| Classification System | Classification Symbols |
| IPC | C12N5/02, 11/04, B01J13/00 |

| Documentation Searched other than Minimum Documentation to the Extent that such Documents are Included in the Fields Searched [5] |
|---|
| |

## III. DOCUMENTS CONSIDERED TO BE RELEVANT [14]

| Category* | Citation of Document, [16] with indication, where appropriate, of the relevant passages [17] | Relevant to Claim No [18] |
|---|---|---|
| P,X | JP, A, 62-25974 (Asahi Chemical Industry Co., Ltd.) 3 February 1987 (03. 02. 87) (Family: none) | 1-4 |
| A | JP, A, 60-174725 (Koken Kabushiki Kaisha) 9 September 1985 (09. 09. 85) (Family: none) | 1 |
| A | JP, A, 59-21388 (Japan Synthetic Rubber Co., Ltd.) 3 February 1984 (03. 02. 84) (Family: none) | 1 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| April 10, 1987 (10. 04. 87) | April 20, 1987 (20. 04. 87) |
| International Searching Authority [1] | Signature of Authorized Officer [10] |
| Japanese Patent Office | |

Form PCT/ISA/210 (second sheet) (October 1981)